# EUROPEAN PATENT APPLICATION

(11) **EP 2 008 536 A1**
(43) Date of publication of application: **31.12.2008**
(21) Application number: 07012788.1
(22) Date of filing: 29.06.2007
(51) Int. Cl.: A41D 11/00, A41D 13/12, A41D 31/00, A61B 5/00, D06P 1/00, G01K 11/12

(54) **Antibacterial garment with thermosensitive colouring**

(71) Applicant: Aloy Font, Juan Manuel, Barcelona (ES)
(72) Inventor: Aloy Font, Juan Manuel, 08459 Sant Pere de Vilamajor Barcelona (ES)
(74) Representative: PROPI, S.L.

(57) **Abstract**

Garment made up of pieces of fabric (1) suitably sewn together, which can take the form of a t-shirt, shirt, pyjamas or any other garment that is not underwear that is worn in direct contact with the user's body, which is made from a fabric comprising between 4% and 8% silver by weight. At least one of the pieces (1) of the garment includes thermo-sensitive pigments or colourings which are applied by conventional methods of dying, painting, printing or transfer to a larger or smaller surface area, said thermo sensitive pigments or colourings being specifically designed to change colour when subjected to a temperature of more than 37.5 °C, thus forming an indicator of the user's body temperature.

## Description

### Object of the invention

The present invention relates to an antibacterial garment of a variable type, with anti-stress, anti-odour and thermal advantages, of the type that is made from one or more pieces of fabric and is preferably designed to be worn by a child; said garment could take the form of a t-shirt, a shirt, pyjamas or any other garment that is not underwear, i.e. that is normally visible when worn.

### Background of the invention.

Until they reach a certain age and develop an adequate immune system, children are often affected by the flu, tonsillitis, bronchitis and other illnesses that generally involve the child running a high temperature. The onset of these symptoms is often sudden, so It is possible that the person or people in charge of looking after the child might not have immediate access to a thermometer to control the child's body temperature, a situation that often occurs outside the home, especially on holiday, at weekends and when travelling.

In such cases, the known method of touching the child's forehead with one's hand or cheek is usually used to find out whether the child has a temperature, which clearly lacks any technical rigour as the resulting sensation can vary according to different factors such as the room temperature or the body temperature of the person who is checking.

Another problem that is also encountered in homes nowadays is the fact that we are surrounded by a large number of electrical systems and magnetic fields, which create a certain amount of static electricity. People are thereby charged with static electricity through their daily movements and, for example, when a child Is held, part of this static electricity is transmitted to him or her, which is not beneficial.

It is also important to keep the child's temperature stable and protect him or her from bacteria and fungi as much as possible in order to prevent potential illnesses.

### Description of the invention.

The antibacterial garment of a variable type that is the object of this invention has structural particularities that make it possible to determine exactly whether the person wearing the garment has a temperature or not because Its colour changes, as well as presenting anti-stress, anti-odour and thermal protection advantages.

The antibacterial garment in question is of the type made from one or more pieces of fabric and it is preferably designed to be worn by children. Said garment can take the form of a t-shirt, shirt, pyjamas or any other garment that is not underwear, i.e. which is normally visible when worn.

According to the invention, the piece or pieces that make up the garment include thermosensitive pigments or colourings, which are applied by conventional methods of dying, painting, printing or transfer to a larger or smaller surface area, said thermosensitive pigments or colourings being specifically designed to change colour when subjected to a temperature of more than 37.5 °C, thus forming an indicator of the user's body temperature.

If the thermosensitive pigments or colourings are applied to specific areas, and not the whole garment, the areas that carry said thermosensitive pigments or colourings will preferably be located on the chest or back, as these are areas that are easily visible, where the garment is in contact with the user's body and the body heat is transmitted directly to said thermosensitive pigments or colourings, thus achieving a greater degree of reliability.

Furthermore, to improve its properties and to protect the child or baby, this fabric includes a thread made with a certain silver content, which can be approximately between 4% and 8% pure silver. As is known, silver is a very good conductor of electricity, thus preventing the accumulation and discharge of static electricity, which cause discomfort and stress. Moreover, the silver-containing thread has antiseptic properties, eliminating bacteria and fungi, and it has anti-odour, anti-stress and thermal effects. It is therefore possible to transmit changes in the child's body temperature more sensitively, thereby protecting him or her.

### Description of the figures.

To complement this description and in order to aid a better understanding of the invention's characteristics, there is a set of illustrative and non-limiting drawings integral to said description, which are as follows:

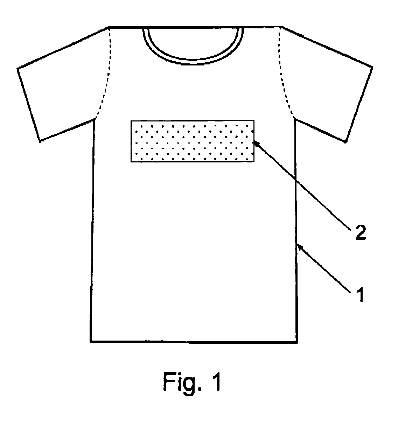

- Figure 1: shows a front elevation view of an embodiment of the garment that is the object of the invention, in this case shown specifically in the form of a t-shirt.

### Preferred embodiment of the invention

As can be observed in the embodiment shown in the figure, the garment, shown here in the form of a t-shirt, is made up of pieces of fabric (1) that form the front, back, sleeves and neckline and are suitably sown together, comprising between 4% and 8% silver by weight. The piece that constitutes the front has an area (2) to which thermosensitive pigments or colourings are applied, which are specifically designed to change colour when subjected to a temperature of more than 37.5 °C.

Said pigments or colourings can be applied to the pieces of fabric (1) by any conventional method of dying, transfer, painting, etc.

Having adequately described the nature of the Invention and an example of a preferred embodiment, it must be noted that the materials, form, size and layout of the described elements may be modified, provided that this does not involve altering the essential characteristics of the invention described in the following claims.

## Claims

1. Antibacterial garment of a variable type, of the type that is made up of pieces of fabric (1) suitably sown together and preferably designed to be worn by a child, said garment being either a t-shirt, shirt, pyjamas or any other garment that is not underwear that can be worn in direct contact with the user's body, **characterised in that** it is made from a fabric comprising between 4% and 8% silver by weight and which has thermosensitive pigments or colourings on at least one of the pieces (1) that make up the garment, which are applied by conventional methods of dying, painting, printing or transfer, on a larger or smaller surface area (2), said thermosensitive pigments or colourings being specifically designed to change colour when subjected to a temperature of more than 37.5 degrees centigrade, thus forming an indicator of the user's temperature.
